# EUROPÄISCHE PATENTANMELDUNG

(11) **EP 3 047 862 A1**
(43) Veröffentlichungstag der Anmeldung: **27.07.2016**
(21) Anmeldenummer: 15151779.4
(22) Anmeldetag: 20.01.2015
(51) Int. Cl.: A61M 1/00, A61L 11/00

(54) **Gelierprodukt für Fluidsammelbehälter**

(71) Anmelder: Medela Holding AG, 6340 Baar (CH)
(72) Erfinder: Bannwart, Lukas, 6340 Baar (CH)
(74) Vertreter: Clerc, Natalia

(57) **Zusammenfassung**

Ein Gelierprodukt zur Verfestigung einer in einem Fluidsammelbehälter (2, 3, 4) abgesaugten Körperflüssigkeit weist einen Innenraum, ein im Innenraum angeordnetes Geliermittel (14) und eine wasserlösliche Abdeckung (1, 10') auf. Die wasserlösliche Abdeckung (1, 10') besteht aus einem textilen Material. Dadurch wird ein verfrühtes Platzen des Gelierprodukts, insbesondere eines Gelierbeutels, verhindert.

## Beschreibung

### TECHNISCHES GEBIET

Die vorliegende Erfindung betrifft ein Gelierprodukt zur Verfestigung einer in einem Fluidsammelbehälter abgesaugten Körperflüssigkeit.

### STAND DER TECHNIK

Zum Absaugen von Körperflüssigkeiten oder Sekreten aus Körperkavitäten oder Wunden werden im medizinischen Bereich, insbesondere in der Thoraxdrainage, stationäre oder mobile Absaugsysteme verwendet. Diese Systeme umfassen jeweils eine Saugquelle, beispielsweise eine Vakuumpumpe oder ein Zentralvakuum, und einen oder mehrere Drainagebehälter. Eine Vakuumleitung verbindet den Drainagebehälter mit der Saugquelle, und eine Drainageleitung führt vom Drainagebehälter zum Patienten. Das angelegte Vakuum erzeugt einen Unterdruck im Behälter, und die abzusaugende Flüssigkeit bzw. das abzusaugende Sekret des Patienten wird in den Drainagebehälter abgesaugt und dort gesammelt. Der Drainagebehälter kann steif ausgebildet sein oder er kann ein weicher, flexibler Beutel sein. Wird ein Beutel verwendet, so kann dieser wiederum in einem steifen, vorzugsweise luftdicht verschliessbaren Aussenbehälter angeordnet sein.

Ein mit abgesaugter Körperflüssigkeit gefüllter Behälter muss fachgerecht entsorgt werden. Dies ist insbesondere dann der Fall, wenn Blut abgesaugt wird. Damit beim Transport und bei der Entsorgung des Behälters keine Flüssigkeit nach aussen gelangen kann und um somit die Infektionsgefahr zu minimieren, wird die Flüssigkeit bereits im Behälter zu einem zähflüssigen Gel verfestigt bzw. verdickt. Behälter mit geliertem Fluid fallen unter eine andere Entsorgungsklasse als Behälter mit nicht verfestigtem Inhalt. Die Entsorgung ist einfacher und billiger.

Ein derartiges Geliergranulat ist beispielsweise aus US 5 595 731 bekannt. Damit das Pulver oder Granulat vor Gebrauch nicht in enge Bereiche des Behälters oder sogar in die Drainageschläuche gelangen kann, ist es üblicherweise zusätzlich verpackt.

US 5 307 819, US 5 234 419, US 5 185 007, US 5 279 602 und WO 2010/006458 offenbaren Trennwände zwischen einem ein Geliermittel beinhaltenden Bereich und dem restlichen Innenraum eines Fluidsammelbehälters, wobei die Trennwände aktiv vom Benützer entfernt werden. Vergisst das Spitalpersonal jedoch, die Trennwand zu entfernen, bleibt das Fluid unverdickt.

US 6 615 862 offenbart eine schwimmende Kugel mit einem darin angeordneten wasserlöslichen Folienbeutel.

In US 5 238 582 ist ein starrer Drainagebehälter gezeigt, in welchem ein Beutel mit einem Geliermittel aufgehängt ist. Die Aussenhülle des Beutels ist entweder eine wasserlösliche Folie oder ein wasserdurchlässiges Gewebe oder ein wasserdurchlässiger Faserstoff.

WO 2009/144486 und WO 2013/030581 offenbaren einen Drainagebeutel, in welchen ein kleiner Beutel oder ein Sachet gefüllt mit Geliergranulat lose eingelegt ist. Der kleine Beutel bzw. das Sachet weist eine Hülle aus wasserlöslichem Papier auf. In anderen Fällen weisen diese Gelierbeutel eine Aussenhülle aus einer wasserlöslichen Folie auf.

Gelierprodukte mit wasserlöslichen Aussenhüllen sind zwar kostengünstig und überall einsetzbar. Sie weisen jedoch den Nachteil auf, dass sie oft bereits vor Gebrauch platzen. Spitäler, welche bei einzelnen neuen Fluidsammelbehältern geplatzte Gelierbeutel finden, neigen dazu, die gesamte Charge an den Hersteller zurückzusenden.

### DARSTELLUNG DER ERFINDUNG

Es ist deshalb eine Aufgabe der Erfindung, ein kostengünstiges Gelierprodukt zu schaffen, bei welchem die Gefahr des Platzens der Aussenhülle minimiert ist.

Diese Aufgabe löst ein Gelierprodukt mit den Merkmalen des Patentanspruchs 1.

Das erfindungsgemässe Gelierprodukt zur Verfestigung einer in einem Fluidsammelbehälter abgesaugten Körperflüssigkeit weist einen Innenraum, ein im Innenraum angeordnetes Geliermittel und eine wasserlösliche Abdeckung auf. Erfindungsgemäss besteht die wasserlösliche Abdeckung aus einem textilen Material.

Es wurde festgestellt, dass Beutel leichter platzen, wenn sie vorgängig einer erhöhten Temperatur und einer erhöhten Luftfeuchtigkeit ausgesetzt worden sind. Dies ist zum Beispiel dann der Fall, wenn der Fluidsammelbehälter mit eingelegtem Gelierprodukt sterilisiert wird, z.B. Ethylenoxid (ETO) - sterilisiert. Bei dieser Art der Sterilisation wird das Gelierprodukt typischerweise Temperaturen von 50°C und einer Luftfeuchtigkeit von 70% ausgesetzt. Das Gelier- bzw. Verfestigungsmittel beginnt zu quellen. Ist die Aussenhülle eine wasserlösliche Folie, so zieht sich diese aufgrund der Temperatur bzw. des Temperaturunterschieds zusammen. Es kann ein harter Beutel entstehen. Bei kälteren Temperaturen, insbesondere im Winter bei Minustemperaturen oder in zu stark gekühlten Räumen, wird die Folie spröde und kann bereits bei einem leichten Schlag platzen.

Dank der erfindungsgemässen Verwendung eines textilen, wasserlöslichen Materials für die Abdeckung lässt sich dies verhindern, da das Material nicht spröde wird. Verfügt das Material über eine genügende Elastizität wird zudem verhindert, dass der Beutel bei leicht gequelltem Geliermittel hart wird.

Das erfinderische Gelierprodukt lässt sich somit vor Gebrauch problemlos gemeinsam mit dem Fluidsammelbehälter sterilisieren.

Der Begriff "textiles Material" wird hier im üblichen Sinne angewendet. Das heisst, es ist damit ein Flächengebilde gemeint, welches aus Fasern und/oder Fäden zusammengefügt wurde. Das textile Material kann beispielsweise ein Vlies oder Filz sein. Es kann aber beispielsweise auch ein Gewebe oder Gewirke sein. Es kann aus einem natürlichen Grundmaterial, einem Kunststoff oder einem Gemisch davon gefertigt sein.

Das Merkmal "wasserlöslich" bedeutet, dass sich das Material durch Kontakt mit Wasser oder einer Wasser enthaltenden Flüssigkeit im bei der Anwendung des Gelierprodukts vorliegenden Temperaturbereich auflöst. Vorzugsweise löst sich die erfindungsgemässe Abdeckung im kalten Wasser oder mindestens bei Kontakt mit Wasser im Raumtemperaturbereich auf. Vorzugsweise ist die erfindungsgemässe Abdeckung in ihrer Materialwahl optimiert, so dass sie sich bei der geplanten Anwendung im vorbestimmten Zeitraum auflöst.

In einer bevorzugten Ausführungsform besteht die wasserlösliche Abdeckung aus PVA (Polyvinylacetat). Vorzugsweise besteht die erfindungsgemässe wasserlösliche Abdeckung aus einem Vlies, insbesondere einem PVA Vlies. Die anderen oben erwähnten Ausführungsformen textiler Materialien lassen sich jedoch auch verwenden.

In einer Ausführungsform besteht das Gelierprodukt lediglich aus dem Geliermittel und der genannten textilen Abdeckung. In einer anderen Ausführungsform ist eine zweite Abdeckung vorhanden, welche aus einem wasserlöslichen und wasserundurchlässigen Material besteht und welche zwischen der wasserlöslichen textilen Abdeckung und dem Geliermittel angeordnet ist. Vorzugsweise besteht diese zweite Abdeckung aus einer Folie, insbesondere aus PVAL (Polyvinylalkohol). Die zweite Abdeckung kann fest mit der ersten Abdeckung verbunden sein, beispielsweise ein zweischichtiges Verbundmaterial bilden, oder sie kann selbsttragend als eigenständiges Element ausgebildet sein.

Das Geliermittel ist üblicherweise ein Granulat bekannter Art, beispielsweise ein quellfähiges, wasserunlösliches vernetztes Polymer. Es lassen sich jedoch auch andere Arten von Verdickungs- oder Verfestigungsmitteln verwenden.

In einem bevorzugten Ausführungsbeispiel umgibt die wasserlösliche Abdeckung das Geliermittel vollständig. Vorzugsweise bildet die Abdeckung einen Beutel, in welchem das Geliermittel gehalten ist. Dieser Beutel lässt sich vorzugsweise lose in einem Fluidsammelbehälter anordnen oder in ihm befestigen. Der Beutel lässt sich in steifen Fluidsammelbehältern wie auch in weichen, flexiblen Fluidsammelbeuteln verwenden.

Ist das Gelierprodukt als Beutel ausgebildet, weist es vorzugsweise mindestens eine Verschlusszone auf zur Bildung eines geschlossenen Hohlraums, welcher den Innenraum für die Aufnahme des Geliermittels bildet. Der Beutel lässt sich aus einer mehrstückigen textilen Abdeckung ausbilden. Vorzugsweise ist die Abdeckung jedoch ein- oder maximal zweistückig.

Die Verschlusszone kann je nach Wahl des Materials und der Form des Gelierprodukts unterschiedlich ausgebildet sein. Vorzugsweise ist sie eine Versiegelung, welche zwei Lagen des Materials der Abdeckung oder eine Lage der Abdeckung mit einem anderen Material, fest verbindet. Die Verbindung muss verhindern, dass pulver- oder granulatförmiges Geliermittel frühzeitig aus dem Innenraum entweichen kann.

Die Verschlusszone kann jedoch alternativ oder zusätzlich eine Naht, eine verklebte Zone oder eine Schweissnaht sein.

Der Gelierbeutel kann eine beliebige Form aufweisen. Bewährt haben sich flache rechteckige oder ovale Formen mit zwei stirnseitigen Verschlusszonen in Querrichtung. Ist der Gelierbeutel aus einem einstückigen Stoff oder Vlies gefertigt, weist er üblicherweise in seiner Längsrichtung eine einzige Verschlusszone auf. Die stirnseitigen Verschlusszonen können geradlinig oder gebogen ausgebildet sein.

In anderen Ausführungsformen weist der Beutel einen runden oder rechteckigen Querschnitt auf, wobei eine Verschlusszone vorhanden ist, welche den gesamten Beutelumfang umläuft und in sich geschlossen ist. Diese Form lässt sich beispielsweise ausbilden, indem zwei annähernd identisch geformte Stoffe oder Vliese übereinander gelegt werden und miteinander versiegelt werden.

In einer anderen Ausführungsform ist der Beutel aus einem nur einmal gefalteten flächigen Material gebildet, wobei eine Verschlusszone vorhanden ist, welche zwei freie Enden aufweist. Die zwischen den Enden liegende Seitenkante des Beutels ist durch die Faltung selber bereits geschlossen ausgebildet.

In einer weiteren Ausführungsform ist der Beutel torusförmig. In einer anderen Ausführungsform ist der Beutel sackförmig ausgebildet und mit einem Clip oder einer Klammer verschlossen.

Das erfindungsgemässe Gelierprodukt ist für Transport und Lagerung vorzugsweise einzeln oder gemeinsam mit identischen Gelierprodukten feuchtigkeitsdicht verpackt, beispielsweise in Folienbeuteln oder in ausgekleideten Kartonschachteln.

Das erfindungsgemässe Gelierprodukt lässt sich als Körper ausbilden, bei welchem lediglich ein Teil seiner Aussenhülle durch die wasserlösliche Abdeckung gebildet ist und der übrige Bereich wasserunlöslich ist. Sein Innenraum lässt sich beispielsweise durch einen starren Hohlzylinder, eine Rückwand und eine der Rückwand beabstandete Vorderwand bilden, wobei die Vorderwand durch die wasserlösliche Abdeckung gebildet ist.

Ein derartiges Gelierprodukt mit lediglich teilweise wasserlöslicher Aussenhülle lässt sich als eigenständiges Element in einen Behälter legen oder befestigen. Es kann jedoch auch ein fester und insbesondere integraler Bestandteil eines Fluidsammelbehälters einer Drainagevorrichtung zum Absaugen einer Körperflüssigkeit sein.

Die Erfindung umfasst deshalb ferner einen Fluidsammelbehälter zur Aufnahme einer abgesaugten Körperflüssigkeit, wobei der Fluidsammelbehälter einen Aufnahmeraum aufweist zur Aufnahme der abgesaugten Körperflüssigkeit und eines derartigen Gelierprodukts, wobei die wasserlösliche Abdeckung eine Trennwand zwischen dem Innenraum des Gelierprodukts und dem Aufnahmeraum bildet und wobei der starre Hohlzylinder und/oder die Rückwand einstückig mit einer das Gelierprodukt umgebenden Wand des Fluidsammelbehälters ausgebildet ist.

Die Erfindung umfasst des Weiteren einen Fluidsammelbehälter zur Aufnahme einer abgesaugten Körperflüssigkeit, wobei der Fluidsammelbehälter einen Aufnahmeraum zur Aufnahme der abgesaugten Körperflüssigkeit und ein oben beschriebenes Gelierprodukt aufweist, wobei das Gelierprodukt in einem in Gebrauchslage des Fluidsammelbehälters unteren Bereich des Aufnahmeraums angeordnet ist. Das Gelierprodukt kann ein lose eingelegter Beutel oder eine lose eingelegte Kapsel mit einem einer bis auf die wasserlösliche Abdeckung steifen Aussenhülle sein. Das Gelierprodukt kann auch am Fluidsammelbehälter befestigt sein, insbesondere kann es Teil der Wandung des Fluidsammelbehälters bilden.

Das erfindungsgemässe Gelierprodukt verhindert ein frühzeitiges Platzen der Aussenhülle.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: einen steifen Drainagefluidsammelbehälter mit einem eingelegten erfindungsgemässen Gelierbeutel;
- Figur 2: einen Drainagefluidsammelbeutel mit flexiblem Beutel und starrem Deckel und mit einem eingelegten erfindungsgemässen Gelierbeutel;
- Figur 3: eine perspektivische Darstellung eines erfindungsgemässen Gelierbeutels in einer ersten Ausführungsform;
- Figur 4: eine Ansicht des Gelierbeutels gemäss Figur 3 von oben;
- Figur 5: einen Teilschnitt durch einen Gelierbeutel in einer zweiten Ausführungsform gemäss der Erfindung;
- Figur 6: eine Ansicht des Gelierbeutels gemäss Figur 5 von oben;
- Figur 7: eine Ansicht eines erfindungsgemässen Gelierbeutels von oben in einer dritten Ausführungsform,
- Figur 8: eine teilweise geschnittene perspektivische Darstellung eines erfindungsgemässen Gelierbeutels in einer vierten Ausführungsform;
- Figur 9: eine teilweise geschnittene perspektivische Darstellung eines erfindungsgemässen Gelierbeutels in einer fünften Ausführungsform;
- Figur 10: eine teilweise geschnittene perspektivische Darstellung eines erfindungsgemässen Gelierbeutels in einer sechsten Ausführungsform;
- Figur 11: eine teilweise geschnittene perspektivische Darstellung eines erfindungsgemässen Gelierbeutels in einer siebten Ausführungsform;
- Figur 12: eine teilweise geschnittene perspektivische Darstellung eines erfindungsgemässen Gelierbeutels in einer achten Ausführungsform;
- Figur 13: eine teilweise geschnittene perspektivische Darstellung eines erfindungsgemässen Gelierbeutels in einer neunten Ausführungsform;
- Figur 14: eine perspektivische Darstellung eines erfindungsgemässen Gelierbeutels in einer zehnten Ausführungsform;
- Figur 15: eine teilweise geschnittene perspektivische Darstellung des Gelierbeutels gemäss Figur 14;
- Figur 16: eine teilweise geschnittene perspektivische Darstellung eines erfindungsgemässen Gelierbeutels in einer elften Ausführungsform;
- Figur 17: einen Längsschnitt durch einen erfindungsgemässen Gelierbeutel in einer zwölften Ausführungsform;
- Figur 18: eine perspektivische Darstellung eines Teils eines steifen Fluidsammelbehälters mit einem erfindungsgemässen Gelierprodukt in einer zwölften Ausführungsform;
- Figur 19: einen Längsschnitt durch den Behälterteil gemäss Figur 18;
- Figur 20: einen Teilschnitt durch den Behälterteil gemäss Figur 18 und
- Figur 21: einen Teilschnitt durch das Gelierprodukt und einem benachbarten Bereich des Behälterteils gemäss Figur 18.

### BESCHREIBUNG BEVORZUGTER AUSFÜHRUNGSFORMEN

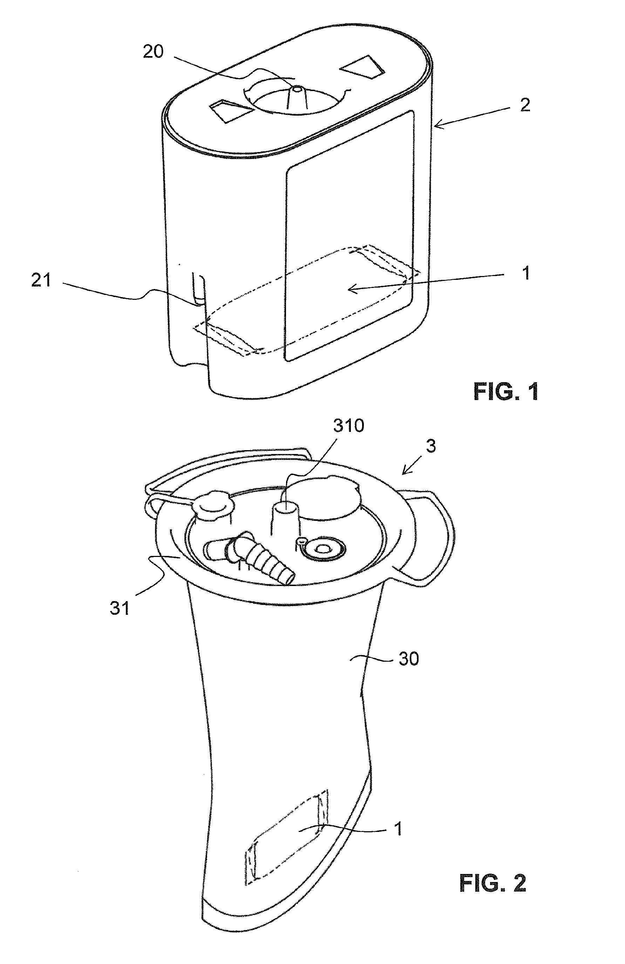

In Figur 1 ist ein bekannter Drainagefluidsammelbehälter 2 dargestellt, wie er beispielsweise gemeinsam mit einer Thoraxdrainagepumpe eingesetzt wird. Er dient zum einmaligen Gebrauch und wird üblicherweise gemeinsam mit den abgepumpten Körperflüssigkeiten des Patienten entsorgt.

Der Behälter 2 ist vorzugsweise aus einem steifen oder starren Kunststoff gefertigt. Er weist einen Drainageanschluss 20 zur Verbindung mit einer zum Patienten führenden Saugleitung sowie einen Sauganschluss 21 zur Verbindung mit einer Vakuumquelle, beispielsweise der Drainagepumpe, auf. Ansonsten ist der Behälter 2 vorzugsweise dicht verschlossen.

Im hohlen Aufnahmeraum des Behälters 2 ist ein erfindungsgemässes Gelierprodukt, hier in Form eines Gelierbeutels 1, eingelegt. Es ist vorzugsweise lose eingelegt, d.h. nicht am Behälter 2 befestigt.

In Figur 2 ist eine Drainagebeutelanordnung 3 bekannter Art dargestellt. Sie weist einen flexiblen Drainagebeutel 30 aus Kunststoff und einen Verschlussdeckel 31 auf. Der Drainagebeutel 30 ist fest und nicht zerstörungsfrei mit dem Verschlussdeckel 31 verbunden. Der Verschlussdeckel 31 weist einen Drainageanschluss 310 zur Verbindung mit einer patientenseitigen Drainageleitung und einen hier in der Abbildung nicht sichtbaren Sauganschluss zur Verbindung mit einer Saugquelle auf. Diese Drainagebeutelanordnung ist üblicherweise bei Gebrauch in einem hier nicht dargestellten starren Aussenbehälter gehalten, wobei der Verschlussdeckel 31 auf dem oberen Rand des Aussenbehälters aufliegt. Auch diese Drainagebeutelanordnung 3 wird nach der erstmaligen Befüllung mit Körperflüssigkeit gemeinsam mit dieser entsorgt. Wie in Figur 2 erkennbar ist, weist auch sie einen lose eingelegten und nicht am Drainagebeutel oder am Deckel befestigten Gelierbeutel 1 auf.

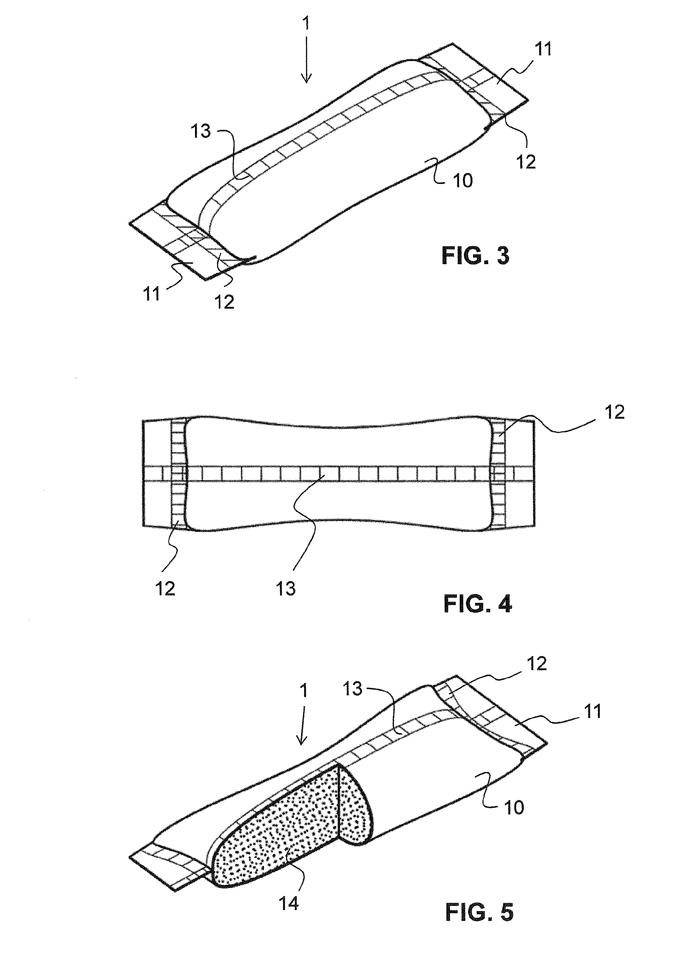

In den Figuren 3 und 4 ist ein erstes Ausführungsbeispiel eines derartigen Gelierbeutels 1, wie er in den Behältern gemäss den Figuren 1 und 2 eingesetzt werden kann, dargestellt. Er weist eine Hülle oder Abdeckung aus einem ein- oder mehrteiligen wasserlöslichen textilen Material auf. Das Material weist Verschlusszonen, hier versiegelte Bereiche auf, so dass ein geschlossener Innenraum zur Aufnahme eines bekannten Geliermittels, insbesondere in Granulatform, gebildet ist. In der hier dargestellten Ausführungsform ist ein einteiliges Vlies, beispielsweise ein PVA Vlies in Längsrichtung zweifach gefaltet und in Längsrichtung versiegelt. Die entsprechende Naht ist mit der Bezugsziffer 13 versehen. An den zwei Stirnseiten ist je eine in Querrichtung verlaufende Versiegelung 12 vorhanden, wobei ein unversiegelter Rand beidseitig vorsteht. Zwischen den zwei stirnseitigen Versiegelungen entsteht so ein Grundkörper 10 mit dem erwähnten Innenraum. Wie in Figur 4 gut erkennbar ist, verlaufen die stirnseitigen Versiegelungen 12 geradlinig. Dieser Gelierbeutel 1 lässt sich relativ einfach aus einer Materialbahn oder einem Materialschlauch automatisch herstellen und befüllen. Anstelle von Versiegelungen lassen sich, wie eingangs erwähnt, auch andere Verbindungsarten einsetzen.

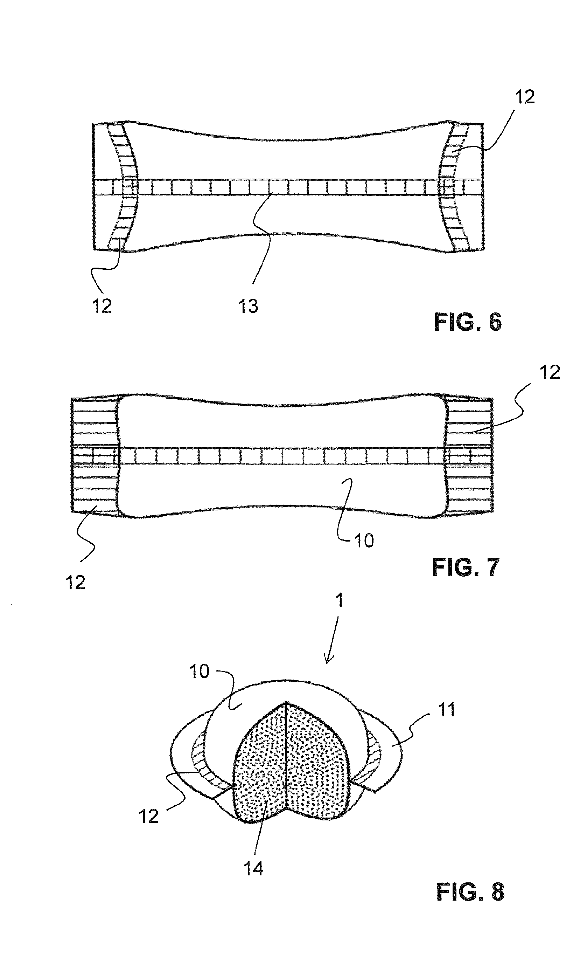

Die Ausführungsform gemäss den Figuren 5 und 6 unterscheidet sich von der vorherigen lediglich in der Form der stirnseitigen Versiegelungen 12. Hier sind sie gebogen ausgebildet. In Figur 5 ist zusätzlich das Geliermittel mit dem Bezugszeichen 14 versehen.

In der Ausführungsform gemäss Figur 7 erstrecken sich die stirnseitigen Versiegelungen 12 über den gesamten Rand bis zum jeweiligen freien Ende.

Der Gelierbeutel 1 gemäss Figur 8 weist einen runden Querschnitt auf, wobei er kugelähnlich mit abgeplatteten Polen ausgebildet ist. Hier ist eine umlaufende und in sich geschlossene Versiegelung 12 vorhanden.

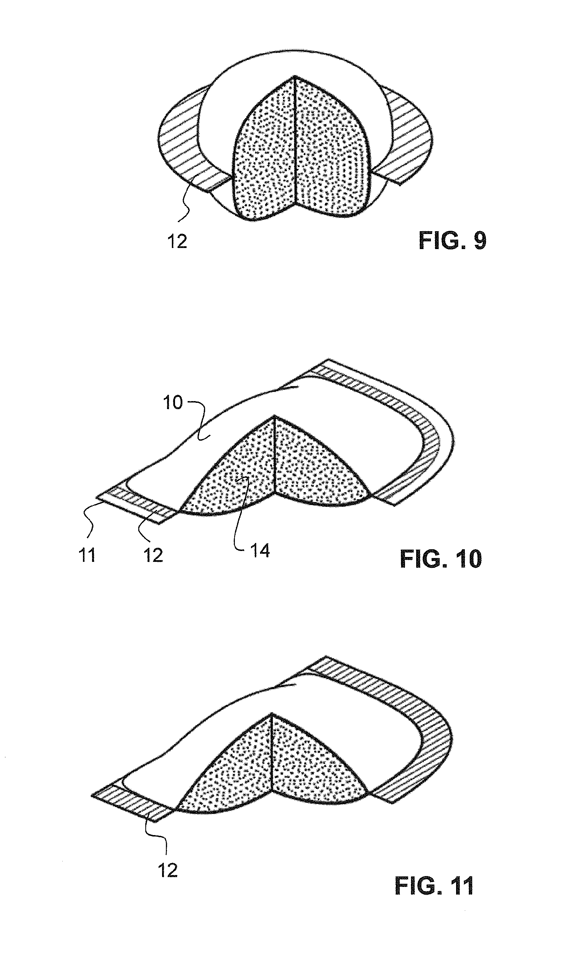

Der Gelierbeutel 1 gemäss Figur 9 entspricht demjenigen der Figur 8, wobei sich hier die Versiegelung 12 wiederum bis zum freien Ende des Randes 11 erstreckt.

Die Ausführungsformen gemäss den Figuren 10 und 11 sind gebildet, indem eine einstückige Abdeckung nur einmal gefaltet wurde, so dass eine Kante des Gelierbeutels 1 durch das Material selber gebildet ist. Die Siegelnaht 12 ist deshalb nicht in sich geschlossen sondern weist zwei freie Enden an dieser Kante auf. Der Unterschied zwischen den zwei Figuren 10 und 11 besteht wiederum in der Breite der Versiegelung 12.

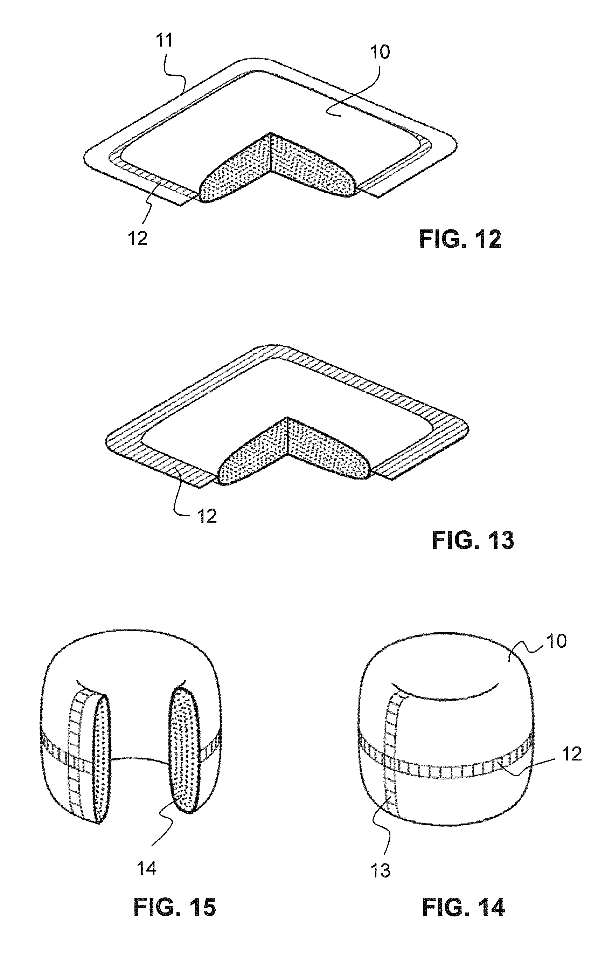

In den Figuren 12 und 13 ist die Form des Querschnitts des Gelierbeutels 1 und des Grundkörpers 10 wiederum rechteckig mit gerundeten Ecken. Hier ist die Versiegelung 12 jedoch umlaufend und in sich geschlossen. Sie kann wiederum beabstandet zu den freien Kanten der Ränder 11 enden oder sich über deren gesamte Breite erstrecken. In diesen Beispielen ist vorzugsweise die Abdeckung zweiteilig, wobei die zwei Teile übereinander gelegt werden und miteinander verbunden werden. Sie können insbesondere miteinander verschweisst, vernäht, versiegelt oder verklebt werden.

In der Ausführungsform gemäss den Beispielen 14 und 15 ist der Gelierbeutel 1 torusförmig ausgebildet.

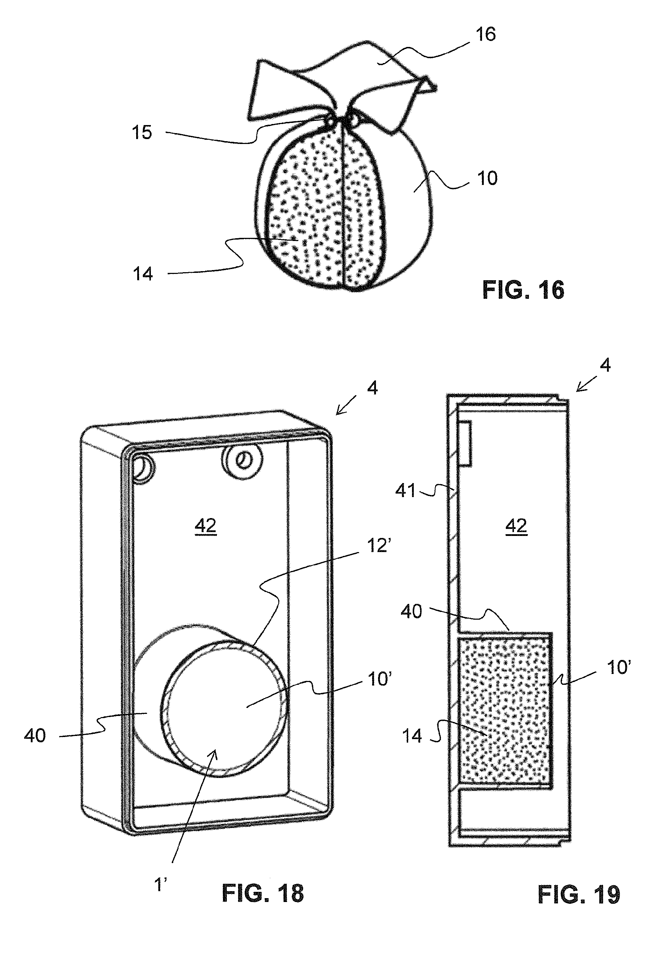

In Figur 16 ist ein Gelierbeutel 1 dargestellt, bei welchem die Abdeckung zusammengefasst und mit einem Clip oder einem anderen Befestigungsmittel zusammengehalten wird. Der restliche Teil der Abdeckung, welcher nicht den Grundkörper 10 bildet, ist in der Figur mit dem Bezugszeichen 16 versehen. Eine Versiegelung oder eine weitere andere Verbindung kann, muss aber nicht vorhanden sein.

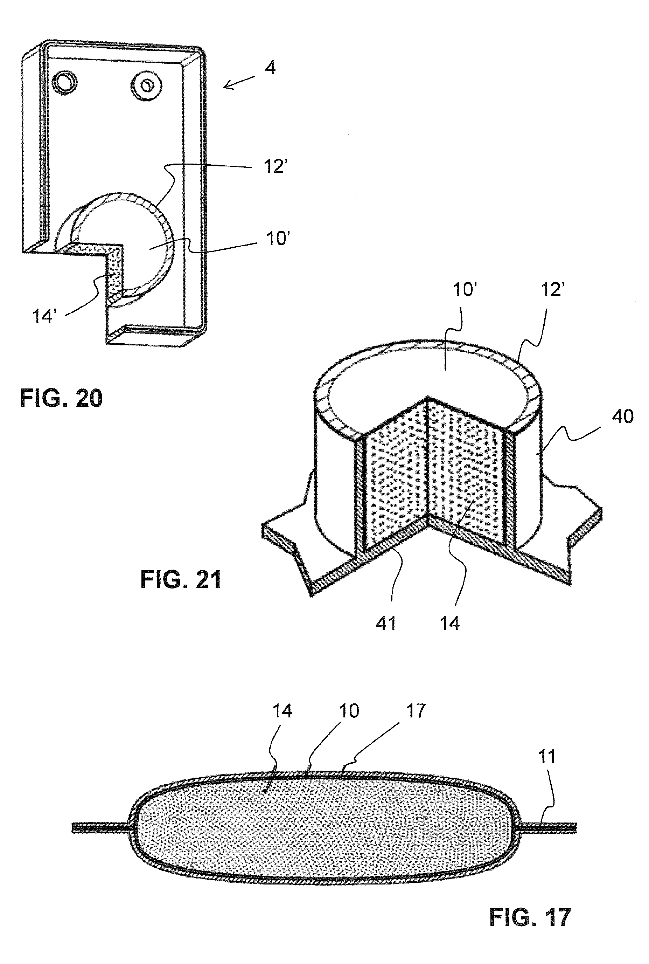

Figur 17 zeigt eine weitere Variante eines erfindungsgemässen Gelierbeutels. Der Beutel ist durch ein zweilagiges Material gebildet, welches das Geliermittel 14 vollständig umgibt. Aussen ist er durch die bereits oben beschriebene textile wasserlösliche erste Abdeckung 10 gebildet. Innen befindet sich eine zweite Abdeckung, welche wasserlöslich, jedoch wasserundurchlässig ist. Vorzugsweise ist die zweite Abdeckung 17 eine Folie. Die erste Abdeckung 10 besteht wiederum vorzugsweise aus einem Vlies, vorzugsweise aus PVA. Die wasserlösliche, aber wasserundurchlässige zweite Abdeckung 17 ist vorzugsweise aus einer PVAL-Folie gefertigt. Beide Abdeckungen 10, 17 bilden gemeinsam den Rand 11. Sie können lose übereinander angeordnet sein, d.h. beide als Beutel ausgebildet sein. Sie können jedoch auch ein gemeinsames Verbundmaterial bilden und zu einem einzigen Beutel geformt sein.

In den Figuren 18 bis 21 ist ein weiteres Gelierprodukt 1' dargestellt. Hier bildet die Abdeckung nicht eine vollständige Umhüllung des Geliermittels 14. Das Gelierprodukt 1' weist einen steifen oder starren Körper auf, welcher durch die wasserlösliche Abdeckung 10' einseitig verschlossen ist. Der Körper ist in diesem Beispiel durch einen Hohlzylinder 40 und eine Rückwand 41 gebildet. Die der Rückwand 41 gegenüberliegende Stirnseite des Hohlzylinders 40 ist von der Abdeckung 10' überdeckt. Die Rückwand 41 ist Teil eines steifen Fluidsammelbehälters 4 einer Drainagevorrichtung. Vorzugsweise ist das Gelierprodukt 1' im unteren Bereich des Fluidsammelbehälters 4 angeordnet.

Der Fluidsammelbehälter 4 entspricht ansonsten beispielsweise dem Behälter wie er in der eingangs erwähnten WO 2010/006458 gezeigt ist. In den Figuren 18 bis 20 ist nur eine Hälfte des Behälters dargestellt. Der Aufnahmeraum 42 für die abgesaugte Körperflüssigkeit ist somit nicht geschlossen dargestellt. Das den Aufnahmeraum 42 und den Behälter verschliessende Gegenstück ist nicht gezeichnet, entspricht jedoch dem Gegenstück gemäss WO 2010/006458.

Wie in Figur 19 erkennbar ist, ist der Hohlzylinder 40 vorzugsweise einstückig mit der Rückwand 40 des Behälters 4 ausgebildet. Sie sind vorzugsweise aus Kunststoff gefertigt. Der Hohlzylinder 40 und der den Zylinder 40 verschliessende Teil der Rückwand 40 können jedoch auch als ein- oder mehrteiliges Einsatzteil ausgebildet sein und fluiddicht in die übrige Rückwand eingesetzt sein. Des Weiteren kann anstelle einer Zylinderform auch eine andere Form, beispielsweise eine Quader- oder Rechteckform, gewählt werden.

Die Verschlusszone, hier eine Versiegelung der Abdeckung 10' auf dem Zylinder 40, ist mit dem Bezugszeichen 12' versehen.

Wie in den Figuren 20 und 21 gut erkennbar ist, ist im Innenraum des Gelierprodukts 1' wiederum das Geliermittel 14, insbesondere in Form eines Granulats, angeordnet. Die Abdeckung 10' hat auch in diesem Beispiel bei Befüllung des Kanisters mit Körperflüssigkeiten Kontakt mit der Flüssigkeit und löst sich entsprechend auf, so dass das Geliermittel 14 freigesetzt wird und die Flüssigkeit verdickt wird.

**BEZUGSZEICHENLISTE**

| | | | |
|---|---|---|---|
| 1 | Gelierbeutel | 2 | Drainagefluidsammelbehälter |
| 1' | Gelierprodukt | 20 | Drainageanschluss |
| 10 | Grundkörper | 21 | Sauganschluss |
| 10' | Abdeckung | | |
| 11 | Rand | 3 | Drainagebeutelanordnung |
| 12 | erste Verschlusszone | 30 | Drainagebeutel |
| 12' | Verschlusszone | 31 | Verschlussdeckel |
| 13 | zweite Verschlusszone | 310 | Drainageanschluss |
| 14 | Geliermittel | | |
| 15 | Verschlussclip | 4 | Drainagebehälter |
| 16 | restlicher Teil der Abdeckung | 40 | Hohlzylinder |
| 17 | zweite Abdeckung | 41 | Rückwand |
| | | 42 | Aufnahmeraum |

## Patentansprüche

1. Gelierprodukt zur Verfestigung einer in einem Fluidsammelbehälter (2, 3, 4) abgesaugten Körperflüssigkeit, wobei das Gelierprodukt einen Innenraum, ein im Innenraum angeordnetes Geliermittel (14) und eine wasserlösliche Abdeckung (1, 10') aufweist, **dadurch gekennzeichnet, dass** die wasserlösliche Abdeckung (1, 10') aus einem textilen Material besteht.

2. Gelierprodukt nach Anspruch 1, wobei die wasserlösliche Abdeckung (10, 10') aus einem Vlies besteht.

3. Gelierprodukt nach einem der Ansprüche 1 oder 2, wobei die wasserlösliche Abdeckung (1, 10') aus PVA (Polyvinylacetat) besteht.

4. Gelierprodukt nach einem der Ansprüche 1 bis 3, wobei eine zweite Abdeckung (17) vorhanden ist, welche aus einem wasserlöslichen und wasserundurchlässigen Material besteht und welche zwischen der wasserlöslichen textilen Abdeckung (10) und dem Geliermittel (14) angeordnet ist.

5. Gelierprodukt nach einem der Ansprüche 1 bis 4, wobei die wasserlösliche textile Abdeckung (1, 10') das Geliermittel (14) vollständig umgibt.

6. Gelierprodukt nach einem der Ansprüche 1 bis 5, wobei die wasserlösliche textile Abdeckung ein Beutel (1) ist.

7. Gelierprodukt nach Anspruch 6, wobei der Beutel (1) eine im Wesentlichen rechteckige Form aufweist mit zwei stirnseitigen Verschlusszonen (12) zur Bildung eines geschlossenen Hohlraums, welcher den Innenraum bildet.

8. Gelierprodukt nach Anspruch 6, wobei der Beutel (1) einen runden oder rechteckigen Querschnitt aufweist, wobei eine Verschlusszone (12) vorhanden ist, welche den gesamten Beutelumfang umläuft und in sich geschlossen ist, wobei die Verschlusszone (12) einen geschlossenen Hohlraum bildet, welcher den Innenraum bildet.

9. Gelierprodukt nach Anspruch 6, wobei der Beutel (1) aus einem nur einmal gefalteten flächigen Material gebildet ist und wobei eine Verschlusszone (12) vorhanden ist, welche zwei freie Enden aufweist, wobei die Verschlusszone (12) einen geschlossenen Hohlraum bildet, welcher den Innenraum bildet.

10. Gelierprodukt nach Anspruch 6, wobei der Beutel (1) torusförmig ist.

11. Gelierprodukt nach Anspruch 6, wobei der Beutel (1) sackförmig ausgebildet ist und mit einem Clip (15) oder einer Klammer verschlossen ist.

12. Gelierprodukt nach einem der Ansprüche 1 bis 5, wobei der Innenraum durch einen starren Hohlzylinder (40), eine Rückwand (41) und eine der Rückwand (41) beabstandete Vorderwand gebildet ist, wobei die Vorderwand durch die wasserlösliche Abdeckung (10') gebildet ist.

13. Gelierprodukt nach Anspruch 12, wobei das Gelierprodukt ein Bestandteil eines Fluidsammelbehälters (4) einer Drainagevorrichtung zum Absaugen einer Körperflüssigkeit ist.

14. Fluidsammelbehälter zur Aufnahme einer abgesaugten Körperflüssigkeit, wobei der Fluidsammelbehälter (4) einen Aufnahmeraum (42) zur Aufnahme der abgesaugten Körperflüssigkeit und das Gelierprodukt gemäss Anspruch 13 aufweist, wobei die wasserlösliche Abdeckung (10') eine Trennwand zwischen dem Innenraum des Gelierprodukts und dem Aufnahmeraum (42) bildet und wobei der starre Hohlzylinder (40) und/oder die Rückwand (41) einstückig mit einer das Gelierprodukt umgebenden Wand des Fluidsammelbehälters (4) ausgebildet ist.

15. Fluidsammelbehälter zur Aufnahme einer abgesaugten Körperflüssigkeit, wobei der Fluidsammelbehälter (2, 3, 4) einen Aufnahmeraum (42) zur Aufnahme der abgesaugten Körperflüssigkeit und das Gelierprodukt gemäss einem der Ansprüche 1 bis 13 aufweist, wobei das Gelierprodukt in einem in Gebrauchslage des Fluidsammelbehälters (2, 3, 4) unteren Bereich des Aufnahmeraums (42) angeordnet ist.
